# EUROPEAN PATENT SPECIFICATION

(11) **EP 2 814 397 B1**
(45) Date of publication and mention of the grant of the patent: **15.01.2020**
(21) Application number: 13749745.9
(22) Date of filing: 13.02.2013
(51) Int. Cl.: A61B 8/00, A61B 8/12, A61B 5/00, A61B 18/14, A61B 18/02, A61B 18/22, A61B 18/18, A61B 90/00, A61B 17/00, A61B 18/00, A61B 18/20

(54) **SYSTEM FOR ASSESSING EFFECTS OF ABLATION THERAPY ON CARDIAC TISSUE USING PHOTOACOUSTICS**
SYSTEM ZUR PRÜFUNG DER AUSWIRKUNGEN EINER ABLATIONSTHERAPIE AN HERZGEWEBE MITTELS PHOTOAKUSTIK
SYSTÈME D'ÉVALUATION DES EFFETS D'UNE THÉRAPIE D'ABLATION SUR UN TISSU CARDIAQUE À L'AIDE DE PHOTOACOUSTIQUE

(30) Priority: 14.02.2012 US 201261598844 P
(43) Date of publication of application: 24.12.2014
(73) Proprietor: St. Jude Medical, Atrial Fibrillation Division, Inc., St. Paul, Minnesota 55117 (US)
(72) Inventor: LUPOTTI, Fermin, Armando, Lake Forest, CA 92630 (US)
(74) Representative: Kramer Barske Schmidtchen Patentanwälte PartG mbB
(86) International application number: PCT/US2013/025890
(87) International publication number: WO 2013/123014

(56) References cited:
- US-A- 5 439 000
- US-A- 5 833 612
- US-A1- 2005 075 574
- US-A1- 2006 122 583
- US-A1- 2007 015 992
- US-A1- 2008 154 257
- US-A1- 2010 125 270
- US-A1- 2010 168 568
- US-A1- 2010 179 432
- US-A1- 2010 280 504
- US-A1- 2011 118 582
- US-A1- 2011 313 417

## Description

### BACKGROUND OF THE INVENTION

### a. Field of the Invention

This invention relates to ablation therapy. In particular, the instant invention relates to a catheter, system and method for assessing the effects of ablation therapy in which an electromagnetic radiation emitter such as an optic fiber is used to deliver electromagnetic radiation to tissue and cause a photoacoustic response from the tissue to thereby allow assessment of characteristics of the tissue.

### b. Background Art

Cardiac arrhythmias (including, but not limited to, atrial fibrillation, atrial flutter, atrial tachycardia and ventricular tachycardia) can create a variety of dangerous conditions including irregular heart rates, loss of synchronous atrioventricular contractions and stasis of blood flow which can lead to a variety of ailments and even death. Atrial fibrillation is the most common arrhythmia, affecting more than six million people in Europe alone. The number of people affected continues to grow rapidly because of an aging population and a strong correlation between atrial fibrillation and increased age. Atrial fibrillation is characterized by uncoordinated atrial activation, thereby disturbing the normal sinus rhythm. Fibrillation waves vary in size, shape, and timing, and result in an irregular, and frequently rapid, ventricular response. Atrial fibrillation is associated with a fivefold increase in stroke risk and has been associated with a number of other cardiovascular and cerebrovascular diseases and strongly affects quality of life.

It is believed that the primary cause of many arrhythmias is stray electrical signals within one or more heart chambers. In the case of atrial fibrillation, the signals originate from an area around the pulmonary veins. Treatments for atrial fibrillation attempt to restore the normal sinus rhythm or control the ventricular rate and include pharmacological treatments and cardioversion. If these types of treatments are unsuccessful, ablation of cardiac tissue can be used to create tissue necrosis and lesions in the tissue. An ablation catheter delivers ablative energy (e.g., radiofrequency energy, light energy, ultrasound, or thermal (cryo or heat based) energy) to the cardiac tissue to create a lesion in the tissue. The lesions disrupt undesirable electrical pathways by isolating areas of tissue and thereby limit or prevent stray electrical signals that lead to arrhythmias.

To be effective, ablation therapy preferably eliminates conductivity in any undesirable electrical pathway and prevents restoration of that conductivity. Excessive ablation, however, increases several risks associated with ablation therapy including perforation of tissue, coagulation of the blood which can result in creation of a thrombus, and tissue or steam pops resulting from the application of heat to water inside the tissue which may cause the water to boil and burst through the tissue wall. Excessive ablation can cause mechanical failure of the cardiac tissue and thereby degrade cardiac function. Perforation of the heart wall can creates a life-threatening complication. The difficulty in determining the appropriate level of ablation therapy results in a long term success rate of only around 70% (which can be raised to 85% through repeated procedures) despite a relatively high cost and with a relatively high rate of complications (around 4.9%).

Because of the negative consequences of delivering too little or too much ablation energy to tissue, it is desirable to continuously monitor the effects of ablation energy on the tissue to assess the effectiveness of ablation therapy. Current monitoring techniques such as measuring tissue elasticity, echogenicity and speed of sound, however, have limited benefits because these techniques have difficulty in identifying lesion boundaries and often rely on sensing mechanisms located far from the site of the ablation.

The inventor herein has recognized a need for a catheter, system and method for assessing the effects of ablation therapy that will minimize and/or eliminate one or more of the above-identified deficiencies.

US 2010/280504 A1 relates to a tissue ablation device including at least one energy emitter and at least one photoacoustic sensor.

US 2010/179432 A1 is directed to the area of intra-vascular ultrasound imaging systems and methods of making and using the systems.

US 2006/122583 A1 relates to method and apparatus for removing tissue from a region of heart muscle to cause revascularization of the muscle.

US 2007/015992 A1 relates to a system for imaging a structural or compositional characteristic of an object comprising at least one coherent, broad range frequency tunable, electromagnetic radiation source to enable generation of an electromagnetic excitation signal, and at least one pulse shaper to control one or more electromagnetic excitation signal characteristic.

### BRIEF SUMMARY OF THE INVENTION

It is desirable to provide a catheter, system and method for assessing the effects of the ablation therapy on tissue in a body.

A system for assessing the effects of ablation therapy on tissue in a body resulting from application of ablation energy to the tissue by an ablation catheter in accordance with one embodiment of the present teachings includes a sheath assembly comprising an elongate deformable shaft having a proximal end and a distal end and an electromagnetic radiation emitter disposed within the shaft. The emitter may comprise an optic fiber in certain embodiments and, in particular, a multi-mode optic fiber. The emitter is configured to emit electromagnetic radiation through an opening in the shaft towards the tissue to thereby cause generation of a photoacoustic wave from the tissue. The system further includes an echographic probe comprising an elongate deformable shaft having a proximal end and a distal end and an ultrasound transducer disposed at the distal end of the shaft of the echographic probe and configured to generate a signal indicative of a characteristic of the tissue responsive to the photoacoustic wave.

A system for assessing the effects of ablation therapy on tissue in a body in accordance with another embodiment of the present teachings includes an echographic probe comprising an elongate deformable shaft having a proximal end and a distal end and an electromagnetic radiation emitter disposed within the shaft. The emitter is configured to emit electromagnetic radiation through an opening in the shaft towards the tissue to thereby cause generation of a photoacoustic wave from the tissue. The echographic probe further includes an ultrasound transducer disposed at the distal end of the shaft and configured to generate a signal indicative of a characteristic of the tissue responsive to the photoacoustic wave.

A system for assessing the effects of ablation therapy on tissue in a body in accordance with another embodiment of the present teachings includes a catheter comprising an elongate deformable shaft having a proximal end and a distal end and an ablation delivery element proximate the distal end of the shaft. The catheter further includes an electromagnetic radiation emitter disposed within the shaft, the emitter configured to emit electromagnetic radiation through an opening in the shaft towards the tissue to thereby cause generation of a photoacoustic wave from the tissue. The catheter further includes an ultrasound transducer disposed at the distal end of the shaft and configured to generate a signal indicative of a characteristic of the tissue responsive to the photoacoustic wave.

A catheter, system and method in accordance with the present teachings are advantageous because they enable improved assessment of the effects of ablation therapy. In particular, the inventive catheter, system and method provide a technique for assessing the formation of lesions both during and after ablation of tissue that allows for assessment in close proximity to the site of the ablation. By locating optic fiber or other light emitter in the ablation catheter or separate sheath assembly, the radiation may be delivered proximate to the ablation site and travels over only a short distance. Further, where the optic fiber or other light emitter is disposed in the ablation catheter, blood between the catheter and tissue may be displaced by fluid irrigation thereby increasing the efficiency of delivery. The resulting photoacoustic wave generated by tissue can be detected by a transducer on either an echographic probe which is typically only several centimeters away or on a combined ablation and echographic probe which will be even closer to the ablation site.

The foregoing and other aspects, features, details, utilities and advantages of the present invention will be apparent from reading the following description and claims, and from reviewing the accompanying drawings.

### BRIEF DESCRIPTION OF THE DRAWINGS

Figure 1 is a diagrammatic view of a system for delivery of ablation therapy to tissue in a body and for assessing effects of the ablation therapy in accordance with one embodiment of the present teachings.
Figure 2 is a diagrammatic view of several components of the system of Figure 1 illustrating one embodiment of the system of Figure 1.
Figure 3 is a diagrammatic view of several components of the system of Figure 1 illustrating another embodiment of the system of Figure 1.
Figure 4 is a diagrammatic view of a system for delivery of ablation therapy to tissue in a body and for assessing effects of the ablation therapy in accordance with another embodiment of the present teachings.
Figure 5 is a diagrammatic view of several components of the system of Figure 4 illustrating one embodiment of the system of Figure 4.
Figure 6 is a diagrammatic view of several components of the system of Figure 4 illustrating another embodiment of the system of Figure 4.
Figure 7 is a cross-sectional view of one embodiment of an echographic probe used in the system of Figure 4.
Figure 8 is a front planar view of the echographic probe of Figure 7.
Figure 9 is a diagrammatic view of a system for delivery of ablation therapy to tissue in a body and for assessing effects of the ablation therapy in accordance with another embodiment of the present teachings.
Figure 10 is a cross-sectional view of one embodiment of a combined echographic and ablation catheter used in the system of Figure 9.
Figures 11-13 are cross-sectional views of the catheter of Figure 10 in accordance with various embodiment of the present teachings
Figure 14 is a cross-sectional view of another embodiment of a combined echographic and ablation catheter used in the system of Figure 9.
Figure 15 is a front planar view of the combined echographic and ablation catheter of Figure 14.
Figure 16 is a flow chart diagram illustrating a method for delivery of ablation therapy to tissue in a body and for assessing effects of the ablation therapy in accordance with one embodiment of the present teachings.

### DETAILED DESCRIPTION OF EMBODIMENTS OF THE INVENTION

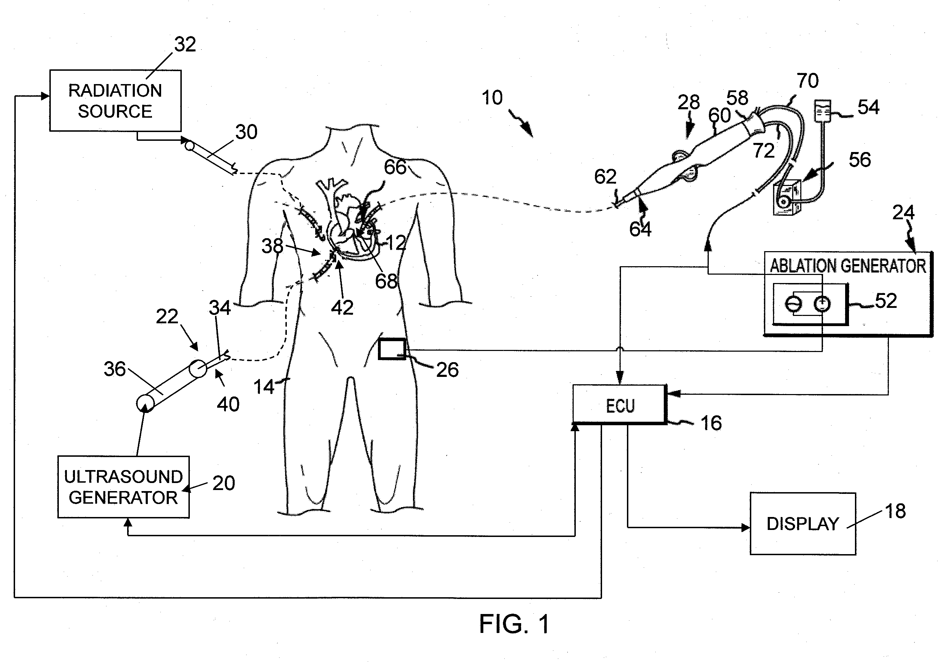

Referring now to the drawings wherein like reference numerals are used to identify identical components in the various views, Figure 1 illustrates a system 10 for delivery of ablation therapy to tissue 12 in a body 14 and for assessing effects of the ablation therapy in accordance with one embodiment of the present teachings. Although the illustrated system relates to diagnosis and treatment of cardiac tissues, it should be understood that the present invention may find application in the diagnosis and treatment of a variety of tissues. System 10 may include an electronic control unit (ECU) 16, a display 18, an ultrasound generator 20, an echographic probe 22, an ablation generator 24, a patch electrode 26, an ablation catheter 28, a sheath assembly 30 for delivery of electromagnetic radiation to tissue 12, and an electromagnetic radiation source 32 that generates electromagnetic radiation that may comprise, for example, visible light, near infrared (NIR), or short wave infrared (SWIR).

ECU 16 is provided to control the operation of ultrasound generator 20, ablation generator 24 and radiation source 30 and process signals generated by echographic probe 22 and ablation catheter 28 to assess deliver of ablation therapy to tissue 12. ECU 16 may be specific to system 10 or may be used in the control of other conventional systems in an electrophysiology (EP) lab including, for example, medical device position, navigation and/or visualization systems, imaging systems, EP monitoring systems and other systems. ECU 16 may comprise a programmable microprocessor or microcontroller or may comprise an application specific integrated circuit (ASIC). ECU 16 may include a central processing unit (CPU) and an input/output (I/O) interface through which ECU 16 may receive a plurality of input signals including signals from probe 22 and catheter 28 and generate a plurality of output signals including those used to control display 18, generators 20, 24 and radiation source 32.

Display 18 is provided to convey information to a physician to assist in diagnosis and treatment of tissue 12. Display 18 may comprise a conventional computer monitor or other display device. Display 18 may comprise a portion of the intracardiac ultrasound console under the trademark "VIEWMATE Z" by St. Jude Medical, Inc. Display 18 may provide a variety of information to the physician including images of tissue 12 or the geometry of the heart generated from signals by probe 22 and catheter 28, EP data associated with tissue 12 and graphs illustrating voltage levels over time for various electrodes on ablation catheter 28. Display 18 may also present a graphical user interface (GUI) to the physician.

Ultrasound generator 20 is provided to control generation of ultrasound signals by echographic probe 22. Generator 20 is conventional in the art and may operate under the control of ECU 16.

Echographic probe 22 provides real time imaging and visualization of tissue 12 and is used to evaluate tissue 12. Echographic probe 22 may comprise an intracardiac echocardiography (ICE) catheter or a trans esophageal (TEE) echoprobe or trans thoracic (TTE) echoprobe. Probe 22 is conventional in the art and may comprise the ICE catheter sold under the trademark "VIEWFLEX PLUS" by St. Jude Medical, Inc. Probe 22 may include an elongate deformable shaft 34, a handle 36, and an ultrasound transducer 38.

Shaft 34 provides structural support to the other components of probe 22 and provides a housing for wires and other conductors extending to transducer 38. Shaft 34 is an elongate, tubular, flexible/deformable member configured for movement within body 14 (Figure 1) and has a proximal end 40 and a distal end 42 (as used herein, "proximal" refers to a direction toward the end of the catheter near the physician, and "distal" refers to a direction away from the physician and (generally) inside the body of a patient). Shaft 34 may be introduced into a blood vessel or other structure within body 14 through a conventional introducer. Shaft 34 may then be steered or guided through body 14 to a desired location such as tissue 12 with a guiding introducer such as the Agilis™ NxT steerable introducer available from St. Jude Medical, Inc. or with guide wires or other means known in the art. Shaft 34 may be made from a conventional polymeric materials such as polyurethane, polyfluoroethylene (PTFE) including PTFE sold under the registered trademark "TEFLON" by E.I. DuPont de Nemours & Co. Corp., polyether block amides, and nylon or thermoplastic elastomers such as the elastomer sold under the registered trademark "PEBAX" by Arkema, Inc. Shaft 34 supports transducer 38 and associated conductors, and possibly additional electronics used for signal processing or conditioning. Shaft 34 further defines one or more lumens configured to house the conductors and steering wires.

Handle 36 provides a location for the physician to hold probe 22 and may further provide means for steering or guide shaft 34 within body 14. For example, handle 36 may include means to actuate various steering wires (not shown) extending through shaft 34 to distal end 42 of shaft 34 to control translation and/or deflection of shaft 34. Handle 36 may also be conventional in the art and it will be understood that the construction of handle 36 may vary. It should be understood that probe 22 may be manipulated manually by a physician using handle 36 or automatically through, for example, robotic controls.

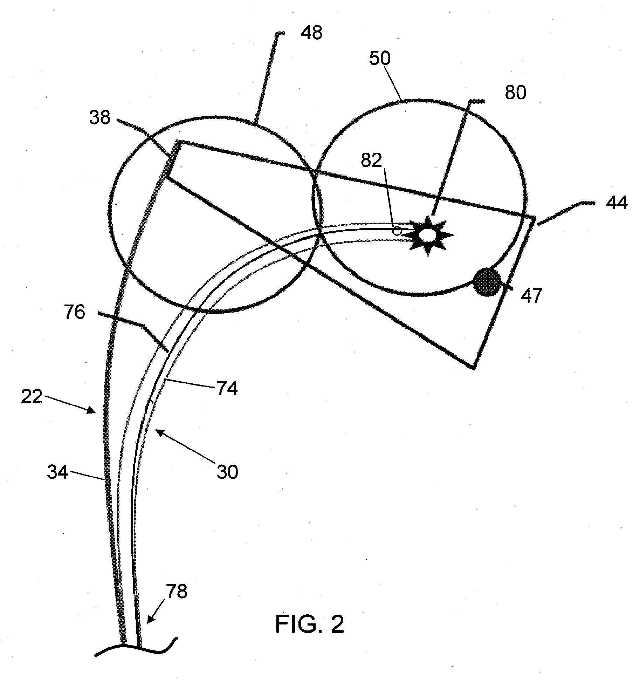

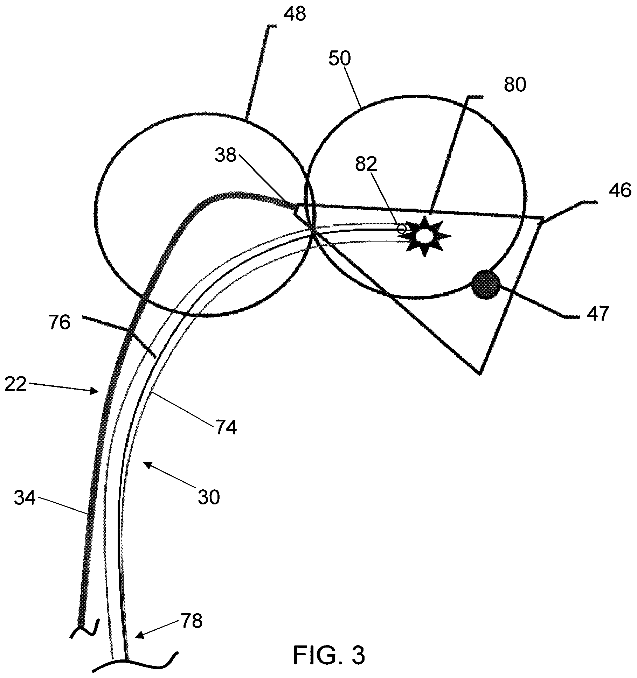

Transducer 38 is provided to convert electrical signals into ultrasound signals transmitted to tissue 12 and to convert reflected ultrasound signals from tissue 12 into electrical signals for processing by ECU 16 and imaging of tissue 12. Transducer 38 is conventional in the art and is disposed at distal end 42 of shaft 34. Referring to Figure 2, transducer 38 may transmit and receive ultrasound signals in a lateral direction (or a direction that is generally perpendicular to a longitudinal axis of shaft 34) creating a field of view or imaging plane 44 that is generally perpendicular to the longitudinal axis of shaft 34. Referring to Figure 3, in another embodiment, transducer 38 may transmit and receive ultrasound signals in a direction that is generally parallel to a longitudinal axis of shaft 34 creating a field of view or imaging plane 46 that is generally parallel to a longitudinal axis of shaft 34. In accordance with one aspect of the present teachings, transducer 38 is configured to generate a signal indicative of a characteristic of tissue 12 in response to one or more photoacoustic waves as described in greater detail hereinbelow. Transducer 38 may alternate between the transmission and receipt of ultrasound signals for regular pulse-echo imaging and the capture of photoacoustic signals for tissue evaluation at predetermined time periods. It should be noted that transducer 38 (and the distal end 42 of shaft 34) may be disposed at some distance from the site of ablation 47. For example, in the case of an ICE catheter, transducer 38 may be disposed in the right atrium 48 of the heart and assess ablation taking place in the left atrium 50 of the heart. It should be understood, however, that transducer 38 may be disposed in any chamber of the heart (including right atrium 48, left atrium 50 or a ventricle) and locations exterior to the heart to assess ablation taking place in any chamber of the heart (including right atrium 48, left atrium 50 or a ventricle) or locations exterior to the heart.

Ablation generator 24 generates, delivers and controls RF energy used by catheter 28. Generator 24 is conventional in the art and may comprise the commercial unit available under the model number IBI-1500T RF Cardiac Ablation Generator, available from Irvine Biomedical, Inc., a St. Jude Medical Company. Generator 24 includes an RF ablation signal source 52 configured to generate an ablation signal that is output across a pair of source connectors: a positive polarity connector which may connect to an electrode on catheter 28; and a negative polarity connector which may be electrically connected by conductors or lead wires to a patch electrode 26 on body 14. It should be understood that the term connectors as used herein does not imply a particular type of physical interface mechanism, but is rather broadly contemplated to represent one or more electrical nodes. Source 52 is configured to generate a signal at a predetermined frequency in accordance with one or more user specified parameters (e.g., power, time, etc.) and under the control of various feedback sensing and control circuitry as is know in the art. Source 52 may generate a signal, for example, with a frequency of about 450 kHz or greater. Generator 24 may also monitor various parameters associated with the ablation procedure including impedance, the temperature at the tip of catheter 28, ablation energy and the position of the catheter 28 and provide feedback to the physician regarding these parameters. The duty cycle of ablation generator 24 may be controlled such that ablation signals are not provided during time periods of pulse-echo imaging and/or receipt of photoacoustic signals by transducer 38. Pulse echo imaging may require a time period of 10 microseconds and the receipt of photoacoustic signals may take about half that time period (in the latter case, transmission of light into the tissue is nearly instantaneous; therefore, the time period for travel of the photoacoustic wave is the only significant contributor to the required time).

Patch electrode 26 provides an RF or navigational signal injection path and/or is used to sense electrical potentials. Electrode 26 may also have additional purposes such as the generation of an electromechanical map or as part of a position sensing and navigation system for catheter 28 or other devices in body 14. Electrode 26 is made from flexible, electrically conductive material and is configured for affixation to body 14 such that electrode 26 is in electrical contact with the patient's skin.

Ablation catheter 28 may be used for examination, diagnosis and treatment of internal body tissues such as tissue 12. In accordance with one embodiment of the invention, catheter 28 comprises an irrigated radio-frequency (RF) ablation catheter. It should be understood, however, that the present invention can be implemented and practiced with other types of ablative energy (e.g., cryoablation, ultrasound, etc.). Catheter 28 may be connected to a fluid source 54 having a biocompatible fluid such as saline through a pump 56 (which may comprise, for example, a fixed rate roller or peristaltic pump or variable volume syringe pump with a gravity feed supply from fluid source 54 as shown) for irrigation. Catheter 28 is also electrically connected to ablation generator 24 for delivery of RF energy. Catheter 28 may include a cable connector or interface 58, a handle 60, a shaft 62 having a proximal end 64 and a distal end 66 and one or more diagnostic or treatment elements supported thereon. Catheter 28 may further include an ablation delivery element 68. Catheter 28 may also include other conventional components not illustrated herein such as a temperature sensor, additional electrodes, one or more position sensors, and corresponding conductors or leads.

Referring again to Figure 1, connector 58 may provide mechanical, fluid and electrical connection(s) for fluid conduit 70 extending from pump 56 and a cable 72 extending from ablation generator 26. Connector 58 is conventional in the art and is disposed at a proximal end of catheter 28.

Handle 60 provides a location for the physician to hold catheter 28 and may further provide means for steering or guiding shaft 62 within body 14. For example, handle 60 may include means to actuate various steering wires (not shown) extending through catheter 28 to distal end 66 of shaft 62 to control translation and/or deflection of shaft 62. Handle 60 may also be conventional in the art and it will be understood that the construction of handle 60 may vary. It should be understood that catheter 28 may be manipulated manually by a physician using handle 60 or automatically through, for example, robotic controls.

Shaft 62 provides structural support to the other components of catheter 28 and may also permit transport, delivery and/or removal of fluids (including irrigation fluids and bodily fluids), medicines, and/or surgical tools or instruments to and from tissue 12. Shaft 62 is an elongate, tubular, flexible/deformable member configured for movement within body 14. Shaft 62 may be introduced into a blood vessel or other structure within body 14 through a conventional introducer. Shaft 62 may then be steered or guided through body 14 to a desired location such as tissue 12 with a guiding introducer such as the Agilis™ NxT steerable introducer available from St. Jude Medical, Inc. or with guide wires or other means known in the art. Shaft 62 may be made from a conventional polymeric materials such as polyurethane, polyfluoroethylene (PTFE) including PTFE sold under the registered trademark "TEFLON" by E.I. DuPont de Nemours & Co. Corp., polyether block amides, and nylon or thermoplastic elastomers such as the elastomer sold under the registered trademark "PEBAX" by Arkema, Inc. Shaft 62 supports steering wires (not shown), ablation delivery element 68 and associated conductors, and possibly additional electronics used for signal processing or conditioning. Shaft 62 further defines one or more lumens configured to house conductors and provide irrigation fluid from fluid source 54 to an external surface of ablation delivery element 68.

Ablation delivery element 68 is provided to deliver ablation energy to tissue 12 to create ablative lesions in tissue 12 and thereby disrupt stray electrical pathways in tissue 12. Element 68 is disposed proximate distal end 66 of shaft 62 (and may be disposed at a distal tip of shaft 62) and may be configured in a variety of ways depending on, among other things, the type of ablative energy to be delivered by element 68. In the illustrated embodiment, element 68 comprises a tip electrode. Element 68 may define a plurality of fluid channels (not shown) in fluid communication with an irrigation lumen in shaft 62 and terminating in outlet ports for the purpose of delivering fluid to an external surface of element 68 in order to cool element 68 and to displace blood between element 68 and tissue 12.

Sheath assembly 30 is provided to deliver electromagnetic radiation to tissue 12 in order to trigger generation of a photoacoustic wave from tissue 12. Ablation creates a thermal lesion in tissue 12 with clearly recognizable optical and structural changes to tissue 12. In particular, the optical absorption of tissue 12 changes. When electromagnetic radiation impinges on tissue 12, energy is absorbed by tissue 12 and adiabatically dissipated. The energy is converted to heat, leading to a transient thermoelastic expansion of tissue 12 which generates photoacoustic waves that can be detected by an ultrasonic transducer such as transducer 38 on probe 22. The contrast in optical absorption between healthy tissue and tissue that has been ablated produces differences in the waves that can be detected by transducer 38. Further, the contrast in optical absorption, and the resulting difference in generated waves, by healthy tissue and ablated tissue, can be used to improve imaging of the lesion. Further information regarding the lesion can also be obtained by correlating the signal with other lesion assessment measures including conductance measurements. Ablation also creates a change in the coloration of tissue 12. This is the result of coagulation necrosis of the tissue 12, shutting down the microperfusion which supplies blood. Healthy tissue may be brown-red, while coagulated tissue is yellow-grey. The discoloration caused by ablation can be detected by photoacoustic imaging and used as a indicator of lesion depth. Referring to Figures 2-3, sheath assembly 30 includes a shaft 74 and an electromagnetic radiation emitter such as optic fiber 76.

Shaft 74 provides structural support to the other components of assembly 30 and may provide a housing for emitter 76 and, in certain embodiments, associated conductors. Shaft 74 may also be used to steer or guide catheters such as ablation catheter 28 and may comprise part of the steerable introducer available under the trademark Agilis™ NxT from St. Jude Medical, Inc. Shaft 74 is an elongate, tubular, flexible/deformable member configured for movement within body 14 (Figure 1) and has a proximal end 78 and a distal end 80. Shaft 74 may be made from a conventional polymeric materials such as polyurethane, polyfluoroethylene (PTFE) including PTFE sold under the registered trademark "TEFLON" by E.I. DuPont de Nemours & Co. Corp., polyether block amides, and nylon or thermoplastic elastomers such as the elastomer sold under the registered trademark "PEBAX" by Arkema, Inc. Shaft 74 supports optic fiber 76 and associated conductors, and possibly additional electronics used for signal processing or conditioning. Shaft 74 further defines one or more lumens configured to house the conductors and steering wires. Optic fiber 76 may be disposed within a central lumen of shaft 74. Alternatively, optic fiber 76 may be disposed in a lumen between radially outer and inner walls of shaft 74. Optic fiber 76 may be movable relative to shaft 74 in a direction parallel to a longitudinal axis of shaft 74.

Optic fiber 76 is provided to deliver electromagnetic radiation to tissue 12 in order to cause tissue 12 to generate a photoacoustic wave that can be sensed by transducer 38 on probe 22. Fiber 76 may be made from various glass compositions (e.g., silica) or plastics (e.g., polymethyl methaacrylate (PMMA) surrounded by fluorinated polymers). Fiber 76 include a core and a cladding with the core having a higher refractive index than the cladding. Fiber 76 may further include a buffer layer and a jacket as is known in the art. Fiber 76 may, for example, comprise any of a variety of common fibers sold by Polymicro Technologies, Inc., Edmund Optics, Inc. or Keyence Corporation. Fiber 76 may comprise a multi-mode optic fiber. Fiber 76 is disposed within shaft 74 and may extend from proximal end 78 to distal end 80 of shaft 74. Fiber 76 itself has a proximal end and a distal end with the distal end terminating proximate an opening (not shown) in the wall of shaft 74. Fiber 76 delivers electromagnetic radiation from the distal end of fiber 76 through the opening to tissue 12. Because the distal end 80 of shaft 74 is disposed proximate the site of ablation, the radiation only has to travel a short distance before impinging on tissue 12 thereby producing efficient delivery of radiation without scattering. Radiation emitted by optic fiber 76 may be unfocused (irradiated omnidirectionally). Alternatively, a focusing lens 82 may be supported by shaft 74 and disposed between the distal end of optic fiber 76 and tissue 12 to focus the radiation in a predefined area and/or provide greater depth of penetration and better target resolution.

Electromagnetic radiation source 32 is provided to generate a set of electromagnetic radiation for delivery to tissue 12 through fiber 76. Source 32 may comprise, for example, a light emitting diode (LED) or laser (e.g., a laser diode). Source 32 may produce a monochromatic or spectral radiation and the radiation may be polarized or unpolarized. Source 32 may generate radiation at various points along the electromagnetic spectrum including, for example, visible light, near infrared (NIR), or short wave infrared (SWIR). In particular, source 32 may generate radiation at different wavelengths (e.g. green and red in the visible spectrum) to provide sufficient contrast for identification of lesion boundaries. The radiation pulses emitted will typically be short (e.g., about 10 nanoseconds). Radiation source 32 may emit radiation in a controlled manner responsive to signals received from ECU 16. In an alternative embodiment, a local electromagnetic radiation source may be supported by shaft 74 near distal end 80 of shaft 74 and function in a manner similar to radiation source 32. Alternatively still, the local electromagnetic radiation source may itself serve as the emitter of electromagnetic radiation and emit electromagnetic radiation directly from shaft 74 towards tissue 12 without transmission through an optic fiber 76.

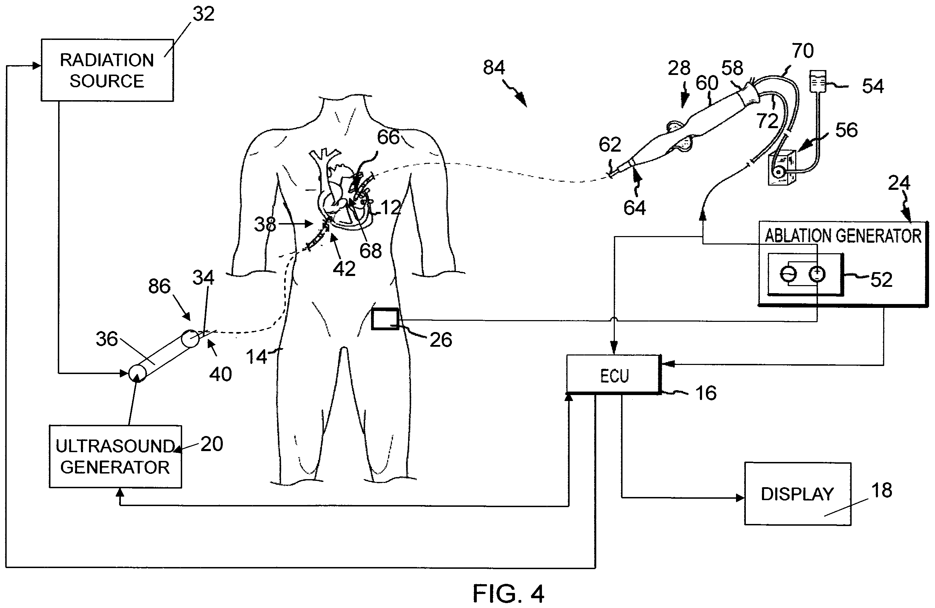

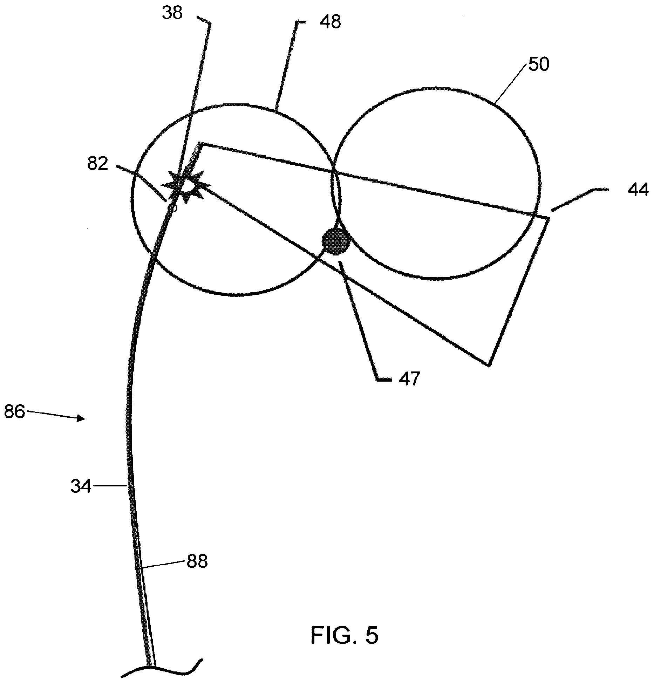

Referring now to Figure 4, a system 84 is illustrated for delivery of ablation therapy to tissue 12 in a body 14 and for assessing effects of the ablation therapy in accordance with another embodiment of the present teachings. System 84 is substantially similar to system 10 and reference to similar components may be made to the descriptions provided hereinabove. Referring now to Figures 5-6, system 84 differs from system 10 in that system 84 integrates the functions of echographic probe 22 and sheath assembly 30 into a single echographic probe 86. Echographic probe 86 is substantially similar to echographic probe 22. Probe 86, however, includes an electromagnetic radiation emitter such as an optic fiber 88. Optic fiber 88 is substantially similar to optic fiber 76 and may extend between the proximal and distal ends 40, 42 of shaft 34 of probe 86 and may be movable relative to shaft 34 in a direction parallel to a longitudinal axis of shaft 34. Fiber 88 delivers electromagnetic radiation from the distal end of fiber 88 through an opening in the wall of shaft 34 to tissue 12. A focusing lens 82 may again be supported by shaft 34 and disposed between the distal end of optic fiber 88 and tissue 12. Fiber 88 may transmit electromagnetic radiation from radiation source 32. In an alternative embodiment, a local electromagnetic radiation source may again be supported by shaft 34 near the distal end of shaft 34 and function in a manner similar to radiation source 32. Alternatively still, the local electromagnetic radiation source may again itself serve as the emitter of electromagnetic radiation and emit electromagnetic radiation directly from shaft 34 towards tissue 12 without transmission through an optic fiber 88.

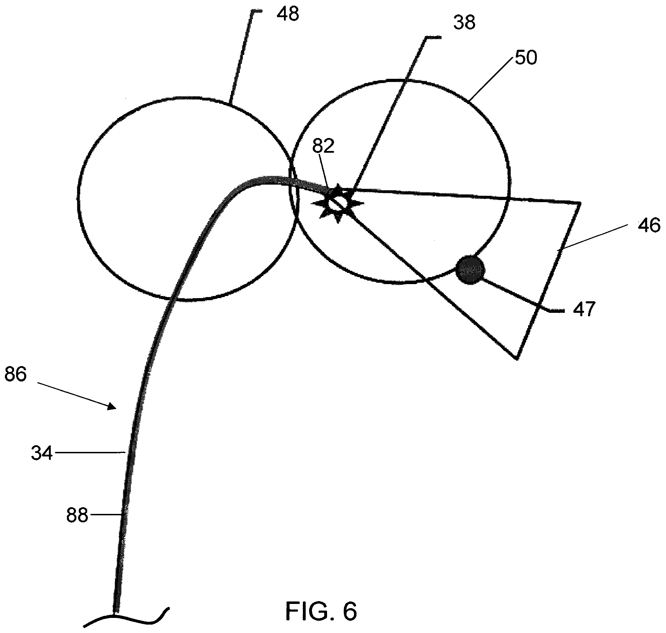

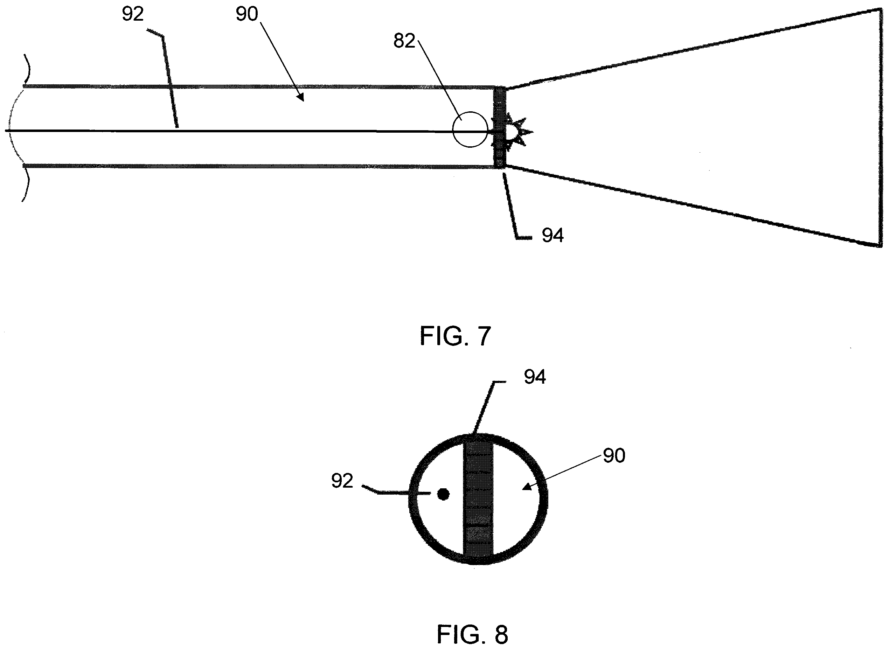

Referring to Figure 5, as with probe 22, the transducer 38 on probe 86 may transmit and receive ultrasound signals in a lateral direction (or a direction that is generally perpendicular to a longitudinal axis of catheter shaft 34) creating a field of view or an imaging plane 44 that is generally perpendicular to the longitudinal axis of shaft 34. Referring to Figure 6, in another embodiment, transducer 38 may transmit and receive ultrasound signals in a direction that is generally parallel to a longitudinal axis of shaft 34 creating a field of view or imaging plane 46 that is generally parallel to the longitudinal axis of shaft 34. Referring now to Figures 7-8, in yet another embodiment, an echographic probe 90 is provided having an optic fiber 92 (similar to fibers 76 or 88) and a two-dimensional transducer array 94 (similar in function to transducer 38) for two-dimensional and/or three-dimensional/four-dimensional (3D/4D) imaging at a distal end of probe 90. Fiber 92 may emit electromagnetic radiation through an opening in the distal end of shaft 34 on either side of array 94.

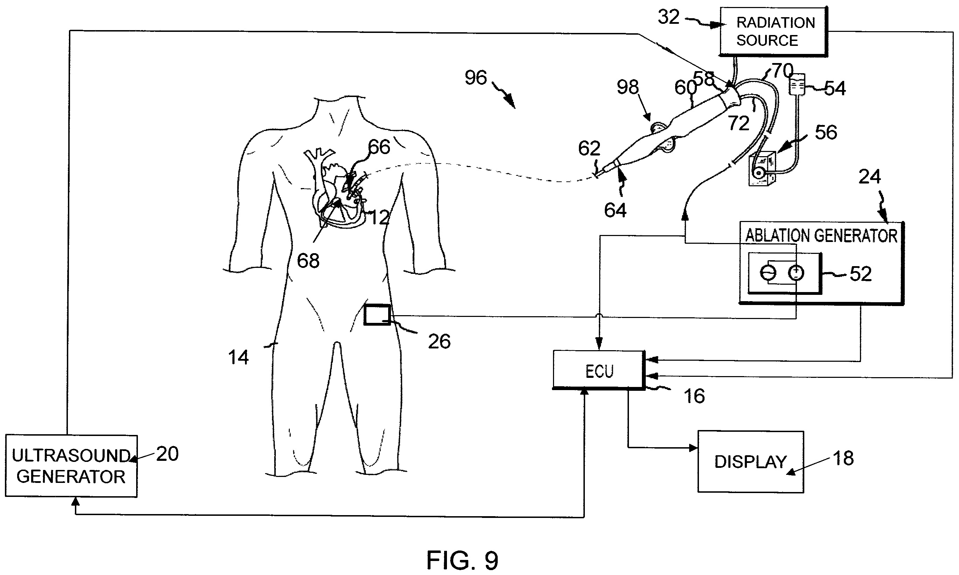

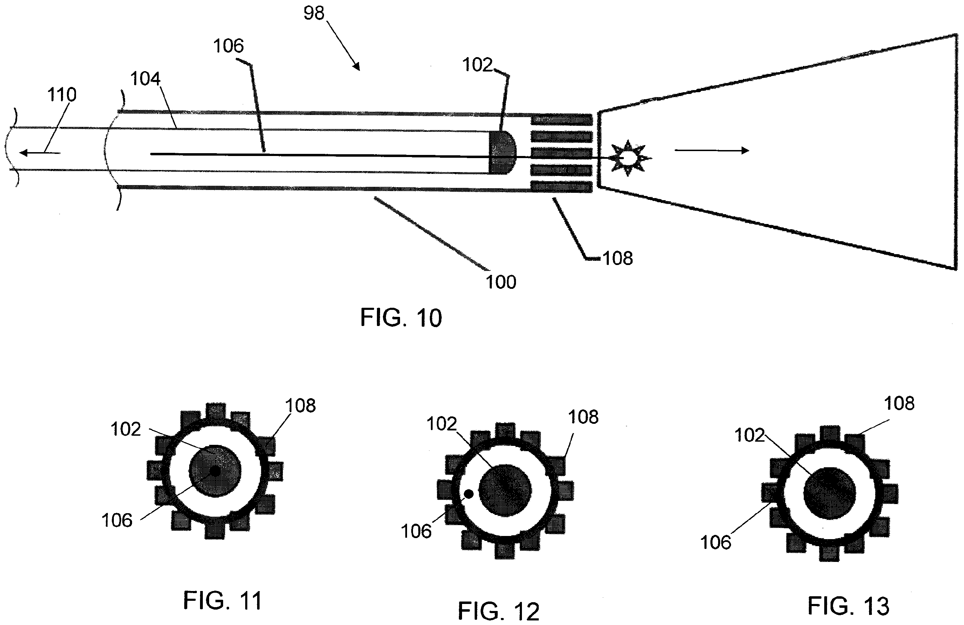

Referring now to Figure 9, a system 96 is illustrated for delivery of ablation therapy to tissue 12 in a body 14 and for assessing effects of the ablation therapy in accordance with another embodiment of the present teachings. System 96 is substantially similar to systems 10 and 84 and reference to similar components may be made to the descriptions provided hereinabove. Referring now to Figures 10-13, system 96 differs from systems 10 and 84 in that system 96 integrates the functions of echographic probe 22, ablation catheter 28 and sheath assembly 30 into a single catheter 98. Referring to Figure 10, catheter 98 may include an elongate, deformable shaft 100, an ablation delivery element 102 which may be supported on another shaft 104, an electromagnetic radiation emitter such as optic fiber 106 and an ultrasound transducer 108.

Shaft 100 may be substantially similar to shaft 34 described hereinabove and may extend from a conventional handle (not shown) similar to handles 36, 60 described hereinabove through which a physician can steer shaft 34. Shaft 100 provides structural support to the other components of catheter 98 and provides a housing for element 102, shaft 104 and optic fiber 106. Shaft 100 is an elongate, tubular, flexible/deformable member configured for movement within body 14 (Figure 1) and has a proximal end and a distal end. Shaft 100 may be introduced into a blood vessel or other structure within body 14 through a conventional introducer. Shaft 100 may then be steered or guided through body 14 to a desired location such as tissue 12 with a guiding introducer such as the Agilis™ NxT steerable introducer available from St. Jude Medical, Inc. (which may itself have a handle for the physician to use in steering the introducer) or with guide wires or other means known in the art. Shaft 100 may be made from conventional polymeric materials such as polyurethane, polyfluoroethylene (PTFE) including PTFE sold under the registered trademark "TEFLON" by E.I. DuPont de Nemours & Co. Corp., polyether block amides, and nylon or thermoplastic elastomers such as the elastomer sold under the registered trademark "PEBAX" by Arkema, Inc. Shaft 100 may further define one or more lumens configured to house conductors and steering wires and provide irrigation fluids.

Ablation delivery element 102 is provided to deliver ablation energy to tissue 12 to create ablative lesions in tissue 12 and thereby disrupt stray electrical pathways in tissue 12. Element 102 is disposed proximate the distal end of shaft 100 and may be configured in a variety of ways depending on, among other things, the type of ablative energy to be delivered by element 102. In the illustrated embodiment, element 102 comprises a tip electrode supported by shaft 104. Element 102 may define a plurality of fluid channels (not shown) in fluid communication with an irrigation lumen in shaft 104 and terminating in outlet ports for the purpose of delivering fluid to an external surface of element 102 in order to cool element 102 and to displace blood between element 102 and tissue 12. In accordance with one aspect of the invention, ablation delivery element 102 is movable relative to shaft 100 in a direction parallel to a longitudinal axis 110 of shaft 100 such that element 102 can be extended and retracted beyond the distal end of shaft 100.

Shaft 104 may be substantially similar to shaft 62 described hereinabove. Shaft 104 provides structural support to ablation delivery member 102 and associate conductors. Shaft 104 is an elongate, tubular, flexible/deformable member configured for movement within body 14 (Figure 1) and has a proximal end and a distal end. Shaft 104 may extend from a conventional handle (not shown) similar to handles 36, 60 described hereinabove through which a physician can steer shaft 104. Shaft 104 may be made from conventional polymeric materials such as polyurethane, polyfluoroethylene (PTFE) including PTFE sold under the registered trademark "TEFLON" by E.I. DuPont de Nemours & Co. Corp., polyether block amides, and nylon or thermoplastic elastomers such as the elastomer sold under the registered trademark "PEBAX" by Arkema, Inc. In accordance with one aspect of the present invention, shaft 104 may be made more flexible than shaft 100 to avoid damage to tissue 12 during contact between ablation delivery element 102 and tissue 12. Shaft 104 further defines one or more lumens configured to house conductors and steering wires and provide irrigation fluids.

Optic fiber 106 is provided to deliver electromagnetic radiation to tissue 12 in order to cause tissue 12 to generate a photoacoustic wave that can be sensed by transducer 108. Fiber 106 may be made from various glass compositions (e.g., silica) or plastics (e.g., polymethyl methaacrylate (PMMA) surrounded by fluorinated polymers). Fiber 106 includes a core and a cladding with the core having a higher refractive index than the cladding. Fiber 106 may further include a buffer layer and a jacket as is known in the art. Fiber 106 may, for example, comprise any of a variety of common fibers sold by Polymicro Technologies, Inc., Edmund Optics, Inc. or Keyence Corporation. Fiber 106 may comprise a multi-mode optic fiber. Fiber 106 is disposed within shaft 100 and may extend from the proximal end to the distal end of shaft 100. Referring to Figures 10-11, in accordance with one embodiment, fiber 106 may be disposed within shaft 104 and may extend through an opening in ablation delivery element 102 and through an aligned opening (not shown) in the front of shaft 100. Fiber 106 may be configured for movement together with ablation delivery element 102 relative to shaft 100 in a direction parallel to axis 110. Alternatively, optic fiber 106 may be configured to move independently of element 102 relative to shaft 100 in a direction parallel to axis 110. Referring to Figures 12-13, in accordance additional embodiments, fiber 106 may alternatively be disposed between shafts 100 and 104 or between the radially outer and inner walls of shaft 100. Fiber 106 may again be configured for movement relative to shaft 100 in a direction parallel to axis 110. Fiber 106 itself has a proximal end and a distal end with the distal end terminating proximate an opening (not shown) in the wall of shaft 100. Fiber 106 delivers electromagnetic radiation from the distal end of fiber 106 through the opening to tissue 12. Because the distal end of shaft 100 is disposed proximate the site of ablation, the radiation only has to travel a short distance before impinging on tissue 12 thereby producing efficient delivery of radiation without scattering. A focusing lens 82 may again be supported by shaft 100 and disposed between the distal end of optic fiber 106 and tissue 12 as discussed hereinabove. Fiber 106 may transmit electromagnetic radiation from radiation source 32. In an alternative embodiment, a local electromagnetic radiation source may again be supported by shaft 100 near the distal end of shaft 100 and function in a manner similar to radiation source 32. Alternatively still, the local electromagnetic radiation source may again itself serve as the emitter of electromagnetic radiation and emit electromagnetic radiation directly from shaft 100 towards tissue 12 without transmission through an optic fiber 106.

Ultrasound transducer 108 is provided to convert electrical signals into ultrasound signals transmitted to tissue 12 and to convert reflected ultrasound signals from tissue 12 into electrical signals for processing by ECU 16 and imaging of tissue 12. Transducer 108 is conventional in the art and is disposed at distal end of shaft 100. In accordance with one embodiment, transducer 108 is arranged about a circumference of shaft 100. In accordance with one aspect of the present teachings, transducer 108 is configured to generate a signal indicative of a characteristic of tissue 12 in response to one or more photoacoustic waves as described in greater detail hereinbelow. Transducer 108 may alternate between the transmission and receipt of ultrasound signals for regular pulse-echo imaging and the capture of photoacoustic signals for tissue evaluation at predetermined time periods.

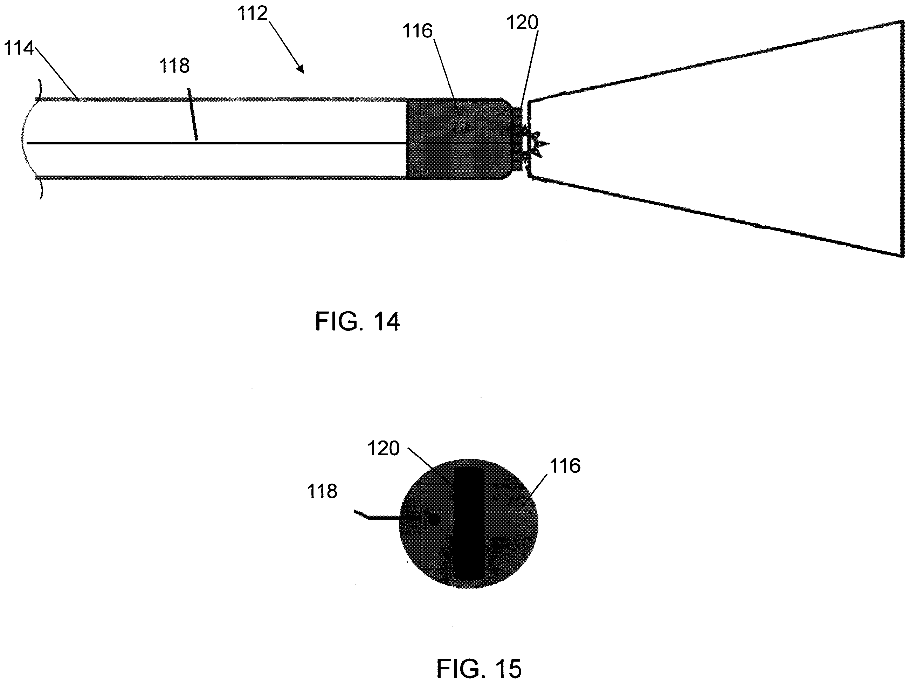

Referring now to Figures 14-15, another catheter 112 is illustrated for use in system 96. Catheter 112 may include an elongate, deformable shaft 114 similar to shaft 104, an ablation delivery element 116 similar to element 102, an electromagnetic radiation emitter such as optic fiber 118 similar to fiber 106. Catheter 112 further includes an ultrasound transducer 120 which is similar in function to transducer 108, but comprises a two-dimensional array transducer formed on or within ablation delivery element 116. Fiber 118 may emit electromagnetic radiation through an opening in delivery element 116 on either side of array transducer 120. As with catheter 98, optic fiber 116 may be movable within shaft 114 in a direction parallel to the longitudinal axis of shaft 114 and may be movable relative to ablation delivery element 116. A focusing lens (not shown) may again be supported by shaft 114 and disposed between the distal end of optic fiber 118 and tissue 12 as discussed hereinabove. Fiber 118 may also again transmit electromagnetic radiation from radiation source 32. In an alternative embodiment, a local electromagnetic radiation source may again be supported by shaft 114 near the distal end of shaft 114 and function in a manner similar to radiation source 32. Alternatively still, the local electromagnetic radiation source may again itself serve as the emitter of electromagnetic radiation and emit electromagnetic radiation directly from shaft 114 towards tissue 12 without transmission through an optic fiber 116.

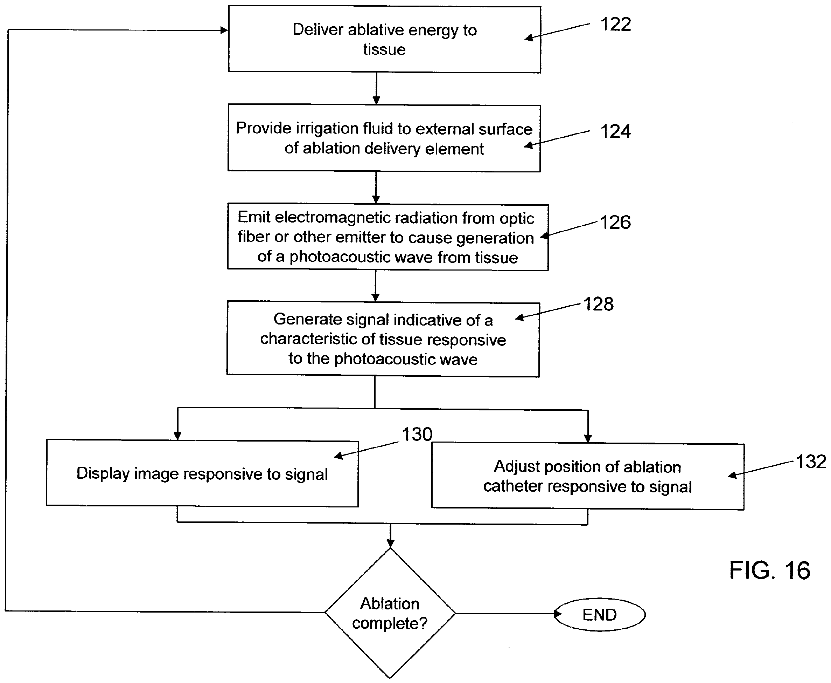

Referring now to Figure 16, a method for delivery of ablation therapy to tissue in a body and for assessing effects of the ablation therapy will be described. The method may begin with the step 122 of delivering ablative energy to tissue 12. Responsive to control by the physician and/or ECU 16, ablation generator 24 may generate signals that cause ablation delivery element 68, 102 or 116 to deliver radio-frequency ablation energy to tissue 12 in a conventional manner. The method may also include the step 124 of providing irrigation fluid to an external surface of the ablation delivery element 68, 102 or 116. It should be understood that step 124 may be performed simultaneously with step 122. It should also be understood that step 124 may be performed continuously even when step 122 is not being performed for the purpose of temperature control and displacement of blood between element 68, 102 or 116 and tissue 12. Irrigation fluid may be delivered from fluid source 54 through an irrigation lumen in shaft 62, 104 or 114 and fluid ports formed in ablation delivery element 68, 102 or 116.

The method may continue with the step 126 of emitting electromagnetic radiation from a distal end of optic fiber 76, 88, 92, 106 or 118 towards tissue 12 to cause generation of one or more photoacoustic waves from tissue 12. ECU 16 may direct ablation generator 24 to cease ablation of tissue 12 while contemporaneously directing radiation source 32 to generate electromagnetic radiation and deliver that radiation to tissue 12 through optic fiber 76, 88, 92, 106 or 118. Ablation creates a thermal lesion in tissue 12 with clearly recognizable optical and structural changes to tissue 12. In particular, the optical absorption of tissue 12 changes. When electromagnetic radiation impinges on tissue 12, energy is absorbed by tissue 12 and adiabatically dissipated. The energy is converted to heat, leading to a transient thermoelastic expansion of tissue 12 which generates photoacoustic waves that can be detected by transducer 38, 94, 108 or 120 on probe 22, 86 or 90 or catheter 98 or 112. The contrast in optical absorption between healthy tissue and tissue that has been ablated produces differences in the waves that can be detected by transducer 38, 94, 108 or 120. Characteristics of the wave (e.g., magnitude, frequency, time of flight, etc.) can be used to determine various characteristics of tissue 12 including lesion depth, size and type and the overall effectiveness of the ablation. Further, the contrast in optical absorption, and the resulting difference in generated waves, by healthy tissue and ablated tissue, can be used to improve imaging of the lesion. Further information regarding the lesion can also be obtained by correlating the signal with other lesion assessment measures including conductance measurements. Ablation also creates a change in the coloration of tissue 12. This is the result of coagulation necrosis of the tissue 12, shutting down the microperfusion which supplies blood. Healthy tissue may be brown-red, while coagulated tissue is yellow-grey. The discoloration caused by ablation can be detected by photoacoustic imaging and used as a indicator of lesion depth.

The method may continue with the step 128 of generating a signal indicative of a characteristic of tissue 12 responsive to the photoacoustic wave. As noted above, transducer 38, 94, 108 or 120 on probe 22, 86 or 90 or catheter 98 or 112 may be used to detect the photoacoustic wave. Probes 22, 86, and 90 are typically a few centimeters from the ablation site (catheters 98 and 112 will be proximate to the site). This distance is too great for light transmission because the blood volume between probes 22, 86 and 90 and the ablation site is too large and too variable to achieve (reproducible) power delivery and imaging. The photoacoustic wave, however, is able to traverse this distance and provide reliable information regarding tissue 12. In response to the photoacoustic wave, transducer 38, 94, 108 or 120 generates a signal which is provided to ECU 16 for processing. The detected characteristic may comprise a depth of a lesion in tissue 12, a size of a lesion in tissue 12, a type of a lesion in tissue 12, a degree of coagulation in tissue 12, a degree of conductivity in tissue 12, or a functionality of a lesion in tissue 12. The characteristic may also comprise a distance of tissue 12 from ablation delivery element 68, 102 or 116. Once the process of causing generation of the photoacoustic wave and the receipt of that wave has occurred, the delivery of ablation energy may resume.

The method may continue with the step 130 of displaying an image of tissue 12 in response to the signal from transducer 38, 94, 108 or 120. In accordance with one embodiment, systems 10, 84, and 96 enable improved imaging of the ablation site based on the ability to contrast healthy tissue and tissue that has been subjected to ablation. The method may also include the step 132 of adjusting a position of catheter 28, 98 or 112 responsive to the signal generated by transducer 38, 94, 108 or 120. Based on information provided by the signal, the physician may manually adjust the position of catheter 28, 98 or 112 including, for example, adjusting a distance between ablation delivery element 68, 102 or 116 and tissue 12 or the orientation of ablation delivery element 68, 102, 116 relative to tissue 12. It should also be understood that this process could occur automatically through robotic control of catheter 28, 98 or 112 responsive to control signals generated by ECU 16 in response to the signals generated by transducer 38, 94, 108 or 120. Further, it should be understood that, while the above-described embodiments focus on a photoacoustic response from tissue 12, information derived from a photoacoustic response of the intervening blood may be also or alternatively be used in adjusting the positions of probe 22, 86 or 90 and catheter 28, 98 or 112. Finally, it should be understood that the steps of the method described herein may be performed in a repeated, iterative fashion until, for example, the physician determines that sufficient ablation has occurred.

A catheter, system and method in accordance with the present teachings are advantageous because they enable improved assessment of the effects of ablation therapy. In particular, the inventive catheter, system and method provide a technique for assessing the formation of lesions both during and after ablation of tissue that allows for assessment in close proximity to the site of the ablation. By locating optic fiber or other light emitter in the ablation catheter or separate sheath assembly, the radiation may be delivered proximate to the ablation site and travels over only a short distance. Further, where the optic fiber or other light emitter is disposed in the ablation catheter, blood between the catheter and tissue may be displaced by fluid irrigation thereby increasing the efficiency of delivery. The resulting photoacoustic wave generated by tissue 12 can be detected by a transducer on either an echocardiograophic probe which is typically only several centimeters away or on a combined ablation and echocardiographic probe which will be even closer to the ablation site.

Although several embodiments of this invention have been described above with a certain degree of particularity, those skilled in the art could make numerous alterations to the disclosed embodiments without departing from the scope of this invention. All directional references (e.g., upper, lower, upward, downward, left, right, leftward, rightward, top, bottom, above, below, vertical, horizontal, clockwise and counterclockwise) are only used for identification purposes to aid the reader's understanding of the present invention, and do not create limitations, particularly as to the position, orientation, or use of the invention. Joinder references (e.g., attached, coupled, connected, and the like) are to be construed broadly and may include intermediate members between a connection of elements and relative movement between elements. As such, joinder references do not necessarily infer that two elements are directly connected and in fixed relation to each other. It is intended that all matter contained in the above description or shown in the accompanying drawings shall be interpreted as illustrative only and not as limiting. Changes in detail or structure may be made without departing from the invention as defined in the appended claims.

Although the various embodiments of the devices have been described herein in connection with certain disclosed embodiments, many modifications and variations to those embodiments may be implemented. For example, particular features, structures, or characteristics described above may be combined in any suitable manner in one or more embodiments. Thus, the particular features, structures, or characteristics illustrated or described in connection with one embodiment may be combined, in whole or in part, with the features structures, or characteristics of one or more other embodiments without limitation unless illogical or non-functional. Also, where materials are disclosed for certain components, other materials may be used. The foregoing description and following claims are intended to cover all such modification and variations.

Any patent, publication, or other disclosure material, in whole or in part, that is said to be incorporated by reference herein is incorporated herein only to the extent that the incorporated materials does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

## Claims

1. A system for assessing effects of ablation therapy on tissue (12) in a body resulting from application of ablation energy to the tissue (12), comprising:
an ablation catheter (28) for applying ablation energy to the tissue (12), comprising:
an elongate deformable shaft (62) having a proximal end and a distal end; and
an ablation delivery element (68) disposed proximate said distal end of said shaft (62) of said ablation catheter (28); and
an echographic probe (22), comprising:
an elongate deformable shaft (34) having a proximal end and a distal end; and,
an electromagnetic radiation emitter disposed within said shaft (34), said emitter configured to emit electromagnetic radiation through an opening in said shaft towards the tissue to thereby cause generation of a photoacoustic wave from the tissue; and,
an ultrasound transducer (38) disposed at the distal end of said shaft and configured to generate an input signal; and
an electronic control unit (ECU) (16) configured to process a plurality of signals including a signal from the ablation catheter (28) and said input signal from the ultrasound transducer (38) and based thereon, to generate an output signal for adjusting position of said ablation catheter (28);
wherein said input signal is indicative of a characteristic of the tissue (12) responsive to the photoacoustic wave.

2. The system of claim 1 wherein said ultrasound transducer (38) is oriented to receive the photoacoustic wave in a direction either substantially perpendicular or substantially parallel to a longitudinal axis of said shaft (34) of said echographic probe (22).

3. The system of any one of claims 1 and 2 wherein said emitter is movable relative to said shaft (34) of said echographic probe (22) in a direction parallel to a longitudinal axis of said shaft (34) of said echographic probe (22).

4. The system of any one of claims 1, 2, and 3 further comprising a focusing lens (82) supported by said shaft (34) of said echographic probe (22) and disposed between said emitter and the tissue (12).

5. The system of any one of claims 1 to 4 wherein said emitter comprises an optic fiber (88) configured to transmit the electromagnetic radiation from an electromagnetic radiation source (32).

6. The system of any one of claims 1 to 5 wherein said optic fiber (88) comprises a multi-mode optic fiber.

7. The system of any one of claims 1 to 6 wherein said ultrasound transducer (38) comprises a two-dimensional ultrasound array (94).

## Patentansprüche

1. System zur Beurteilung der Effizienz einer Ablationstherapie für Gewebe (12) in einem Körper als Folge der Anwendung einer Ablationsenergie auf das Gewebe (12), mit:
einem Ablationskatheter (28) zum Anlegen der Ablationsenergie an das Gewebe (12), der aufweist:
einen länglichen, verformbaren Schaft (62) mit einem proximalen Ende und einem distalen Ende; und
ein Ablationslieferelement (68), das proximal zu dem distalen Ende des Schafts (62) des Ablationskatheter (28) vorgesehen ist; und
einer echographischen Sonde (22), die aufweist:
einen länglichen, verformbaren Schaft (34) mit einem proximalen Ende und einem distalen Ende; und
einen elektromagnetischen Strahlungsemitter, der innerhalb des Schafts (34) vorgesehen ist, wobei der Emitter konfiguriert ist zum Ausgeben einer elektromagnetischen Strahlung durch eine Öffnung in dem Schaft in Richtung des Gewebes, um dadurch die Erzeugung einer fotoakustischen Welle von dem Gewebe zu verursachen; und
einen Ultraschalltransducer (38), der an dem distalen Ende des Schafts vorgesehen und konfiguriert ist zur Erzeugung eines Eingangssignals; und
einer elektronischen Steuerungseinheit ECU (16), die konfiguriert ist zur Verarbeitung einer Mehrzahl von Signalen, die ein Signal von dem Ablationskatheter (28) und das Eingangssignal von dem Ultraschalltransducer (38) aufweisen, und basierend darauf zur Erzeugung eines Ausgangssignals zum Einstellen einer Position des Ablationskatheters (28);
wobei das Eingangssignal kennzeichnend ist für eine Charakteristik des Gewebes (12) in Antwort auf die fotoakustische Welle.

2. System nach Anspruch 1, bei dem der Ultraschalltransducer (38) orientiert ist zum Empfangen der fotoakustischen Welle in Richtung entweder im Wesentlichen senkrecht oder im Wesentlichen parallel zu einer Längsachse des Schafts (34) der echographischen Sonde (22).

3. System nach einem der Ansprüche 1 und 2, bei dem der Emitter relativ zu dem Schaft (34) der echographischen Sonde (22) in Richtung parallel zu der Längsachse des Schafts (34) der echographischen Sonde (22) bewegbar ist.

4. System nach einem der Ansprüche 1, 2 und 3, ferner mit einer Fokussierungslinse (82), die von dem Schaft (34) der echographischen Sonde (22) getragen wird, und die zwischen dem Emitter und dem Gewebe (12) vorgesehen ist.

5. System nach einem der Ansprüche 1 bis 4, bei dem der Emitter eine optische Faser (88) aufweist, die konfiguriert ist zum Übertragen der elektromagnetischen Strahlung von einer elektromagnetischen Strahlungsquelle (32).

6. System nach einem der Ansprüche 1 bis 5, bei dem die optische Faser (88) eine optische Multimodusfaser ist.

7. System nach einem der Ansprüche 1 bis 6, bei dem der Ultraschalltransducer (38) eine zweidimensionale Ultraschallanordnung (94) aufweist.

## Revendications

1. Système d'évaluation des effets d'une thérapie par ablation sur un tissu (12) dans un corps résultant de l'application d'énergie d'ablation au tissu (12), comprenant :
un cathéter d'ablation (28) pour appliquer une énergie d'ablation au tissu (12), comprenant :
une tige déformable allongée (62) ayant une extrémité proximale et une extrémité distale ; et
un élément de délivrance d'ablation (68) disposé à proximité de ladite extrémité distale de ladite tige (62) dudit cathéter d'ablation (28) ; et
une sonde échographique (22), comprenant :
une tige déformable allongée (34) ayant une extrémité proximale et une extrémité distale ; et,
un émetteur de rayonnement électromagnétique disposé à l'intérieur de ladite tige (34), ledit émetteur étant configuré pour émettre un rayonnement électromagnétique à travers une ouverture dans ladite tige vers le tissu pour provoquer ainsi la génération d'une onde photo acoustique à partir du tissu ; et,
un transducteur à ultrasons (38) disposé à l'extrémité distale de ladite tige et configuré pour générer un signal d'entrée ; et
une unité de commande électronique (ECU) (16) configurée pour traiter une pluralité de signaux comprenant un signal provenant du cathéter d'ablation (28) et ledit signal d'entrée provenant du transducteur à ultrasons (38) et, sur la base de celui-ci, pour générer un signal de sortie pour ajuster la position dudit cathéter d'ablation (28) ;
où ledit signal d'entrée est indicatif d'une caractéristique du tissu (12) sensible à l'onde photo acoustique.

2. Système selon la revendication 1, dans lequel ledit transducteur à ultrasons (38) est orienté pour recevoir l'onde photo acoustique dans une direction sensiblement perpendiculaire ou sensiblement parallèle à un axe longitudinal de ladite tige (34) de ladite sonde échographique (22).

3. Système selon l'une quelconque des revendications 1 et 2, dans lequel ledit émetteur est mobile par rapport à ladite tige (34) de ladite sonde échographique (22) dans une direction parallèle à un axe longitudinal de ladite tige (34) de ladite sonde échographique (22).

4. Système selon l'une quelconque des revendications 1, 2 et 3 comprenant en outre une lentille de focalisation (82) supportée par ladite tige (34) de ladite sonde échographique (22) et disposée entre ledit émetteur et le tissu (12).

5. Système selon l'une quelconque des revendications 1 à 4, dans lequel ledit émetteur comprend une fibre optique (88) configurée pour transmettre le rayonnement électromagnétique d'une source de rayonnement électromagnétique (32).

6. Système selon l'une quelconque des revendications 1 à 5, dans lequel ladite fibre optique (88) comprend une fibre optique multimode.

7. Système selon l'une quelconque des revendications 1 à 6, dans lequel ledit transducteur à ultrasons (38) comprend un réseau ultrasonore bidimensionnel (94).
